# EUROPEAN PATENT APPLICATION

(11) **EP 1 780 265 A1**
(43) Date of publication of application: **02.05.2007**
(21) Application number: 05023258.6
(22) Date of filing: 25.10.2005
(51) Int. Cl.: C12N 5/06

(54) **Differentiation of stem cells into chondrocytes**

(71) Applicant: Stiftung CAESAR, 53175 Bonn (DE)
(72) Inventor: Degistrici, Özer, Dr., 53177 Bonn (DE); Thie, Michael, Dr., 53175 Bonn (DE); Schindel, Marina, Dr., 53757 St. Augustin-Hangelar (DE)
(74) Representative: Schrooten, Rolf

(57) **Abstract**

The invention relates to a method for achieving differentiation of stem or progenitor cells derived from tooth tissue into chondrocytes *in vitro,* wherein said stem or progenitor cells are isolated from a sleeve-like extrapulpal soft tissue of a tooth or wisdom tooth, and wherein said stem cells are stimulated using Transforming Growth Factor ß (TGFß). The invention further relates to differentiated cells and chondrocytes derived from a sleeve-like extrapulpal soft tissue as well as uses of these cells.

## Description

### Field of the invention

The invention relates to a method for achieving differentiation of stem or progenitor cells derived from tooth tissue into chondrocytes *in vitro.* The invention further relates to differentiated cells and chondrocytes derived from certain tooth tissue as well as uses of these cells.

### Background of the invention

Several million people throughout the world suffer from arthropathy or cartilage malfunction caused by various reasons. As cartilage covers the surfaces of joints it is the slide which ensures a frictionless relative movement of interacting bones. Cartilage spreads the pressure acting on the joint surface onto the cancellous bone. To this end, cartilage encompasses a thin layer of specialized cells which produce and maintain the matrix forming the protective layer. It is a specific characteristic of cartilage that its metabolic activity is low due to the absence of blood vessels. This leads to a minor regeneration capability and thus cartilage is vulnerable to degenerative alterations. About 80 - 90 % of people at the age of 65 or more develop massive degenerative alterations in the coating of joint surfaces, in particular in lower extremities, leading to arthrosis. But to date there have been no long term strategies for treating cartilage malfunction or damage.

Tissue engineering is a promising new technology for biological reconstruction of joint surfaces. In tissue engineering autologous, cartilage-like grafts are produced on appropriate three-dimensional carriers using *in vitro* cell culture technology. Besides the choice of the carrier material, generating a sufficient number of vital, phenotypically stable cartilage cells from a small biopsy is the major problem in this approach. To date treatment of massive cartilage malfunction is not yet possible because chondrocytes loose their cartilage-specific properties during the necessary expansion.

Stem cells are self-renewing cells that divide to give rise to a cell with an identical developmental potential and/or with a more restricted developmental potential, i.e. stem cells can divide asymmetrically to yield a stem cell and a more specialised cell which has lost some developmental potential. A stem cell has the ability to divide for indefinite periods, at least through many cycles, and often throughout the whole life of the organism. Given specific signals in the development of the organism, stem cells can differentiate into many different cell types that make up the organism. That is, stem cells have the potential to develop into mature cells that have characteristic shapes and specialised functions, such as bone cells, muscle cells, skin cells or nerve cells. Unipotent stem cells produce a single type of differentiated cell, whereas pluripotent stem cells produce most cell types of the body.

Stem cells derived from the inner cell mass of blastocysts (Embryonic Stem cells = ES cells) can give rise to cells that can form a wide range of cell types, such as the mesoderm, endoderm and ectoderm of an embryo, and thus have the highest potential for differentiation. However, to date ES cells are not usable due to severe problems in the molecular control of cell differentiation. Furthermore, ethical concerns have been raised about the use of embryonic stem cells.

Thus, non-embryonic stem cells, i.e. somatic stem cells (Adult Stem cells = AS cells), are now of increasing importance for developing innovative therapeutic strategies. An adult (somatic) stem cell (AS cell) is an undifferentiated cell that is situated in a differentiated tissue but can renew itself and become specialised to yield all of the specialised cell types of the tissue from which it originated, and possibly other specialised cells. For example, stem cells harvested from bone marrow have been used in therapy in cases of leukemia, lymphomas and other life-threatening diseases. Recent studies have demonstrated that mesenchymal stem cells derived from bone marrow may be able to generate both skeletal muscle and neurons. Thus, there is evidence that AS cells derived from a specific type of tissue can generate mature, fully functional cells of another type and that after exposure to a new environment they may be able to populate other tissues and possibly differentiate into other cell types. However, surgical removal of bone marrow from the iliac crest or rip is a sophisticated and risky operation technique so that it is desirable to use other types of tissue as a source for somatic stem cells.

### Prior art

WO 03/066840 A2 concerns a method for isolating non-embryonic stem cells derived from the dental follicle (dental sac) of tooth germs. The resulting stem cells are stated to be pluripotent and capable of differentiation into specialised cells of endodermal, ectodermal and mesodermal lineages.

WO 2004/085630 A1 discloses a method for isolating mesenchymal precursor cells from perivascular compartments of vascularised tissues. Isolation is achieved by selecting the cells using the perivascular marker 3G5. It is suggested to use the precursor cells for regenerating that type of tissue from which they have been sourced. It is further anticipated that the precursor cells may also be useful for regenerating tissue in another tissue type. The proposed source tissues for isolating the precursor cells comprise tooth tissue, in particular dental pulp, while the range of cell types that may be generated from the precursor cells comprises osteoblasts and chondrocytes. However, it has been merely demonstrated that mesenchymal precursor cells derived from dental pulp are capable of regenerating dental/pulp microenvironments. In a further approach, differentiation of adipose-derived precursor cells into chondrocytes and cartilage, respectively, was accomplished by stimulation with TGFβ3 as described by Pittenger et al. (1999).

### Summary of the invention

It is the problem to be solved by the present invention to provide a method for achieving differentiation of stem or progenitor cells into chondrocytes *in vitro,* which is effective and uses a source tissue that is easily available.

The problem is solved by a method for achieving differentiation of stem or progenitor cells into chondrocytes *in vitro,* wherein said stem or progenitor cells are isolated from a sleeve-like extrapulpal soft tissue of a tooth or wisdom tooth, and wherein said stem cells are stimulated using Transforming Growth Factor β (TGFβ).

According to the invention ectomesenchymal soft tissue is used, which is located extrapulpally in immediate vicinity of a developing, immature tooth or wisdom tooth. This extrapulpal soft tissue surrounds the developing tooth like a sleeve and comprises stem cells which give rise to different tissues and/or portions of the mature tooth. Beneficial tooth tissue to be used as source material for the method according to the invention comprises dental follicle (dental sac including operculum) as well as tissue residing underneath the dental papilla in immediate vicinity of the apical side of a developing tooth. In the stage of development between the appearance of the bony alveolar fundus and the end of the formation of the root, no dental follicle tissue is located at the apical side of the immature tooth but lateral follicle can be observed. That is, the follicular cells are either dissipated to form cementum, periodontal ligament and/or alveolar bone or migrated towards the crown. However, when a non-erupted, immature tooth is surgically removed a pad-like tissue is observed, which is attached to the dental papilla and/or the root at the apical side of the developing tooth. This valuable tissue is a white, pad-like soft tissue structure, which approximately has the size of a lentil or a bean, depending on the developmental stage. The pad-like tissue can only be detected in a defined, specific developmental stage in an early phase of root formation. That is, identifying and separating the pad-like tissue is only possible from the appearance of the bony alveolar fundus to the end of the formation of the root of the tooth. The apical pad-like tissue comprises at least multipotent stem cells which can differentiate as ectomesenchymal stem cells (mesenchymal cells derived from the ectodermal neural crest). Since said pad-like tissue and also dental follicle are by-products of surgically removed non-erupted teeth, these tissues are easily available without limitation. Additionally, the quantity of useful singularised cells is sufficient for transplantation or other medical purposes. Moreover, the resulting stem cells are at least multipotent and can be used for producing functional cells and tissues of several types, even types that are different from tooth tissue. Thus, in a preferred embodiment of the invention, stem cells derived from the pad-like tissue as well as from dental follicle are used as source material for the method according to the invention.

In an advantageous embodiment of the invention, only stem cells are stimulated, which express at least surface marker CD90, CD49e and/or CD73. Moreover, only stem cells should be used for stimulation which express surface marker CD13 at low level. It is another selection criterion that the stem cells to be stimulated do not exhibit any expression of surface marker CD31 and/or CD45.

The stem cells to be stimulated are expanded in a cell culture medium comprising a minimal medium that contains at least 10 % fetal calf serum (FCS) and 1 g/l glucose. Said stem cells are washed prior to stimulation with a cell culture medium comprising a minimal medium that contains at least 4.5 g/l glucose, 1 % ITS+ premix, 35 µg/ml L-ascorbate-2-phosphate, 1 mol/l sodium pyruvate and 10⁻⁷ mol/l dexamethasone. Finally, the said stem cells may be stimulated with TGFβ1 or preferably TGFβ3. Stimulation of the stem cells is preferably accomplished in a cell culture medium comprising a minimal medium that contains at least 4.5 g/l glucose, 1 % ITS+ premix, 35 µg/ml L-ascorbate-2-phosphate, 1 mol/l sodium pyruvate and 10⁻⁷ mol/l dexamethasone, preferably for at least 25 days, particularly 28 days.

In a preferred embodiment of the invention, the tooth or wisdom tooth from which the soft tissue is sourced is a mammalian tooth, preferably a human tooth. If the origin of the stem cells is human tooth tissue, the method according to the invention is the first step of promising cell therapies for humans, e.g. in respect of degenerative disorders, such as arthrosis.

The present invention also concerns differentiated former stem cells stimulated by the method according to the invention, preferably chondrocytes produced by this method. Thus, the method according to the invention may be employed to achieve differentiated cells for regeneration or replacement of cartilage.

The problem is also solved by a chondrocyte derived from a sleeve-like extrapulpal soft tissue of a tooth or wisdom tooth and/or dental follicle of a tooth or wisdom tooth and/or a pad-like tissue located in immediate vicinity of the apical side of an immature, developing tooth or wisdom tooth underneath the dental papilla, wherein said tooth or wisdom tooth is in a stage of development between the appearance of the bony alveolar fundus and the end of the formation of the root of said tooth or wisdom tooth.

In a preferred embodiment of the invention, the chondrocyte according to the invention is a mammalian cell, preferably a human cell, and hence a promising source for successful cell therapy for humans or other mammals.

Any cell culture or cell structure comprising at least one differentiated cell or chondrocyte according to the invention and pharmaceutical compositions comprising at least one differentiated cell or chondrocyte according to the invention are subject to the invention as well. The cell cultures or cell structures which include at least one differentiated cell or chondrocyte according to the invention can give rise to functional cartilage. The pharmaceutical compositions according to the invention are based on at least one differentiated cell or chondrocyte according to the invention and allow for treating various diseases or disorders resulting from cartilage degeneration. Possible therapies include repair or replacement of chondrocytes and cartilage, respectively, by administration and/or transplantation of differentiated cells or chondrocytes according to the invention, or tissues or molecules derived therefrom. A cell therapy can be accomplished by administering to a patient, preferably a mammal, in particular a human-being, an amount of the differentiated cells or chondrocytes according to the invention, which is sufficient to cause a therapeutic effect. The pharmaceutical compositions according to the invention may further comprise usual auxiliary and/or carrier substances.

The differentiated cell according to the invention, the chondrocyte according to the invention, the cell culture or cell structure according to the invention, and the pharmaceutical composition according to the invention, each can be advantageously used for repairing or replacing damaged or destroyed cartilage in vivo.

The differentiated cell according to the invention, the chondrocyte according to the invention, the cell culture or cell structure according to the invention, and the pharmaceutical composition according to the invention, each can be advantageously used for treating arthrosis.

The invention also comprises the use of at least one: stem cell derived from a sleeve-like extrapulpal soft tissue of a tooth or wisdom tooth and the use of at least one stem cell derived from dental follicle of a tooth or wisdom tooth for obtaining differentiated chondrocytes as well as the use of at least one stem cell derived from a pad-like tissue located in immediate vicinity of the apical side of an immature, developing tooth or wisdom tooth underneath the dental papilla in a development stage between the appearance of the bony alveolar fundus and the end of the formation of the root of said tooth or wisdom tooth for obtaining differentiated chondrocytes.

The invention is described below in detail with reference to the drawings.

### Brief description of the drawings

In the figures:
- Figure 1: shows a sagittal section through an unerupted developing human tooth;
- Figure 2: shows a tissue section through the apical soft tissue of a wisdom tooth showing histological details of the apical dental region, H.E. staining;
- Figure 3: represents a micrograph of colony-forming, fibroblastic stem cells;
- Figure 4: represents a bar chart of a quantitative s-GAG analysis after chondrogenic stimulation with Transforming Growth Factor β (TGFβ1 and TGFβ3), amount of s-GAG [µg/sample], control: without TGFβ, samples 176 and 177: extrapulpal soft tissue, BM-MSC: mesenchymal stem cells from bone marrow;
- Figure 5: represents micrographs of stimulated cells according to the invention after chondrogenic stimulation, the sample being stained with toluidine blue,
a) 1 day stimulation
b) 28 days stimulation.

### Various and preferred embodiments of the invention

According to the invention stem or progenitor cells derived from ectomesenchymal soft tissue of teeth or wisdom teeth were isolated and analysed by a set of cell surface markers. Suitable cells were stimulated to differentiate into chondrocytes under cell culture conditions using special media for differentiation. Determination of chondrocytes was accomplished by detecting the production of collagen type II and typical proteoglycans.

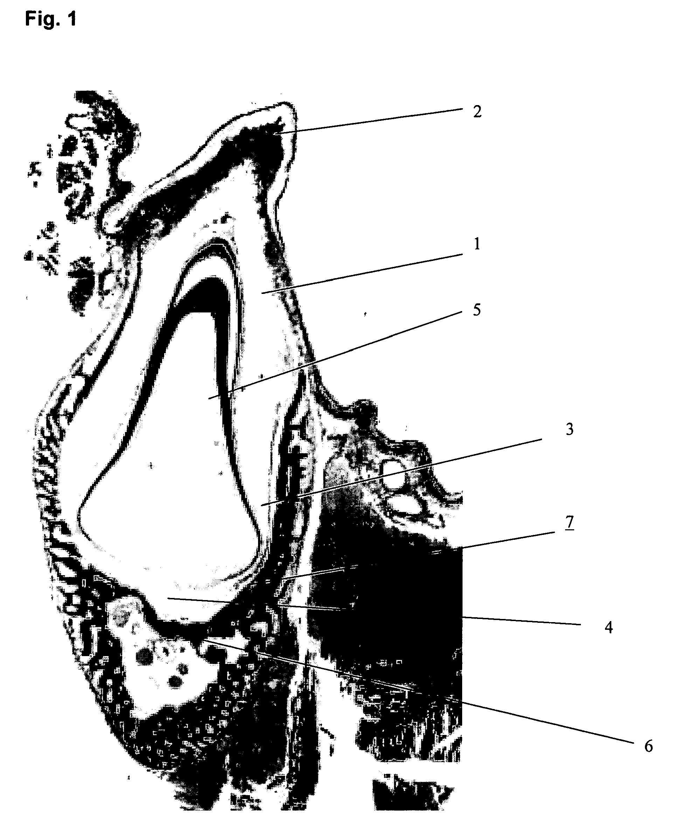

Figure 1 represents a sagittal section through a developing unerupted human tooth 1. At the occlusal side of the tooth 1 the operculum 2 including mucosa can be observed. The dental follicle 3 surrounds the lateral sides of the tooth 1. The pad-like tissue 4 is located at the apical side of the tooth 1 between the dental papilla 5 and the alveolar bone 6. Thus, at its apical side the pad-like tissue 4 contacts the occlusal surface of the alveolar bone 6. Both the dental follicle 3 and the pad-like tissue 4 are part of the extrapulpal soft tissue 7 surrounding the tooth 1 like a sleeve. Preferably, immature teeth are used as source material for the extrapulpal soft tissue. It is an advantage of this material that it is easily available in a significant number, particularly with children or adolescent persons.

After typical dissection and osteotomy, the bony cover above the alveolar compartment was removed and subsequently the tooth was carefully removed. Preferably, the pad-like tissue 4 located at the apical side of the tooth underneath the dental papilla 5 is precisely separated from the dental papilla 5 and, if necessary, from the developing root, e.g. by cutting along the macroscopically visible border between the dental papilla 5 and the pad-like tissue 4 with a scalpel. The pad-like tissue 4 is a white, jelly-like tissue which approximately has the size of a lentil or a bean, depending on the stage of development of the developing tooth. This tissue comprises undifferentiated ectomesenchymal (neuroectodermal) stem cells. However, stem cells derived from dental follicle may also be used as source material for the method according to the invention.

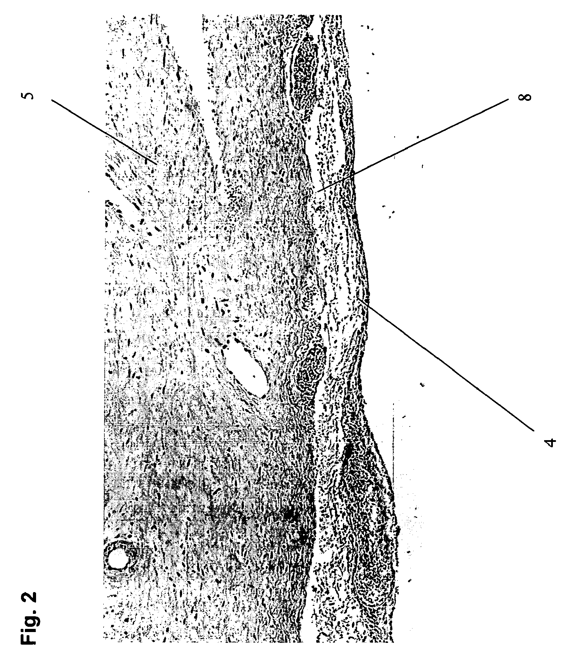

Figure 2 shows a tissue section through the apical soft tissue of a surgically removed immature wisdom tooth (3^{rd} molar) showing histological details of the apical dental region by H.E. staining. This histological analysis shows that the pad-like tissue 4 is a connective tissue with a low level of collagen fibres. While no adipose tissue can be observed in the pad-like tissue 4, it shows capillary blood vessels and nerve fibres. Histologically, condensed connective tissue or loose, vascularized connective tissue can be observed in the border region 8 between the dental papilla 5 and the occlusal side of the pad-like tissue 4. This border region 8 approximately corresponds to the location of the epithelial diaphragm which is a process of Hertwig's epithelial borderline that turns from lateral to central and relates to root formation with multi-root teeth in later stages of development. In contrast to the dental papilla 5, which has a high cell density and includes numerous blood vessels and nerve fibres; the apical pad-like tissue 4 is characterised by a low cell density, a low matrix level and a lower level of blood vessels and nerve fibres. The cells of the pad-like tissue 4 are flat and spindle-shaped and aligned parallel to the surface of the tissue in the apical border zone. In contrast, the cells of the dental papilla 5 are star-shaped cells having spherical nuclei, which are randomly distributed in the tissue.

After separation, the soft tissue was digested with collagenase/dispase in serum-free medium for 1 h at 37°C. After disintegrating the tissue, the singularised cells were placed in a T25 cell culture flask in a medium comprising DMEM low glucose (1 g/l), 10 % fetal calf serum (FCS) and 1 % penicillin/streptomcin/glutamine, and cultured at 37°C in a humidified atmosphere of 5% CO₂. After 7 to 14 days of incubation, formation of single colonies of fibroblastoid cells occurred.

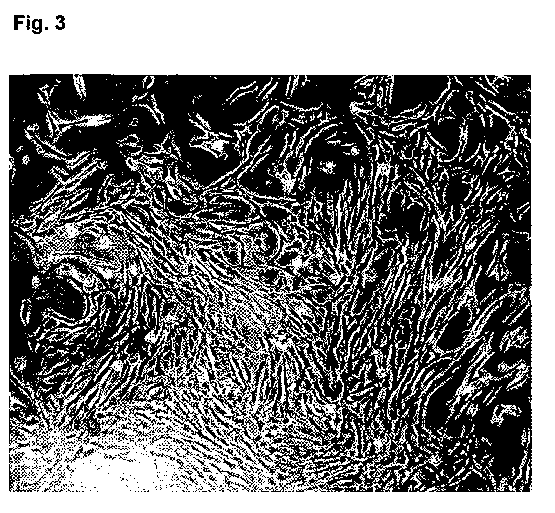

Figure 3 represents a micrograph of isolated stem cells. When the cells reached approximately 50 to 60 % confluency they were passaged to a new culture flask (5,000 cells per cm²) and cultured further. The cells of the pad-like tissue show a heterogeneous appearance which changes in the course of time until, in the end, fibroblast-like cells dominate the culture forming a confluent multilayer.

In the second passage, the cells were analysed by flow cytometric analysis (FACS) for expression of various cell surface markers. The cells expressed CD90, CD49e and CD73 at high level, CD13 at low level, and did not express any CD31 or CD45.

In order to achieve differentiation of the cells into chondrocytes a 3D-culture model was used. To this end, the cells were expanded in a culture medium comprising 10% fetal calf serum (FCS), DMEM low glucose (1 g/l), 1 % penicillin/streptomycin/ glutamine. The cells were then transferred into a 96-well plate (2x10⁵ cells per well) and washed once in a basic medium (DMEM high glucose (4.5 g/l), 1 % ITS+ premix, 35 µg/ml L-ascorbate-2-phosphate, 1 mol/l sodium pyruvate, 10⁻⁷ mol/l dexamethasone, 1% penicillin/streptomycin/glutamine). The cells were further cultured in stimulation medium (basic medium including 10 ng/ml TGFβ1 or TGFβ2) for 28 days. Medium was changed three times a week. After 28 days, the samples are either fixed in para-formaldehyde for histologic analysis or digested for biochemical analysis using papain. For molecular-biological analysis the cells were suspended in lysis buffer.

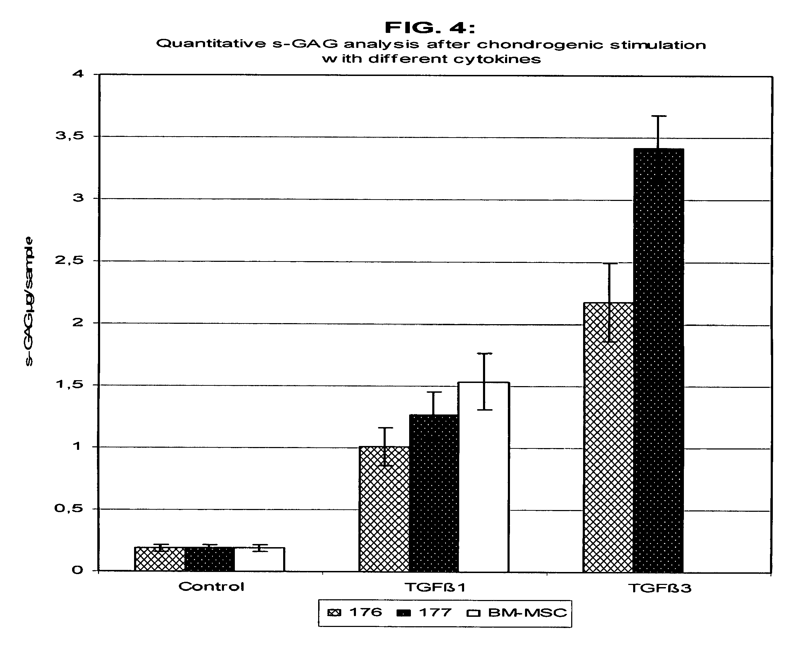

Figure 4 represents a bar chart of a biochemical determination of sulphated glycosaminoglycan (s-GAG) expression during chondrogenic differentiation of mesenchymal stem cells derived from soft tissue of wisdom tooth compared to stem cells derived from human bone marrow. It is demonstrated that expression of s-GAG is significantly increased compared to start conditions. Synthesis and secretion of s-GAG which is a component of cartilage represents an evidence for differentiation into cartilage-forming chondrocytes.

Production of collagen type II is deemed to be a further evidence for chondrogenic differentiation. Table 1 shows a determination of collagen type II - mRNA by RT-PCR. The results represented by Table 1 indicate that after 28 days of stimulation with TGFβ the stimulated cells expressed collagen type II at high level (Sample No. 176 and hBMSC) or at least at low level (Sample No. 177). As a result, successful differentiation of the stem cells derived from tooth tissue could be demonstated.

**Table 1: RT-PCR analysis of collagen type II expression in chondrogenically differentiated stem cells derived from ectomesenchymal soft tissue of human teeth**

| Sample | 176 | 176 | 177 | 177 | hBMSC | hBMSC |
|---|---|---|---|---|---|---|
| Day | 0 | 28 | 0 | 28 | 0 | 28 |
| TGFβ1 | - | ++ | - | + | - | ++ |
| TGFβ3 | - | ++ | - | + | n.d. | n.d. |

| | | | | | | |
|---|---|---|---|---|---|---|
| - = no expression, + = low-level expression, ++ = high-level expression, hBMSC = human bone marrow stem cells | | | | | | |

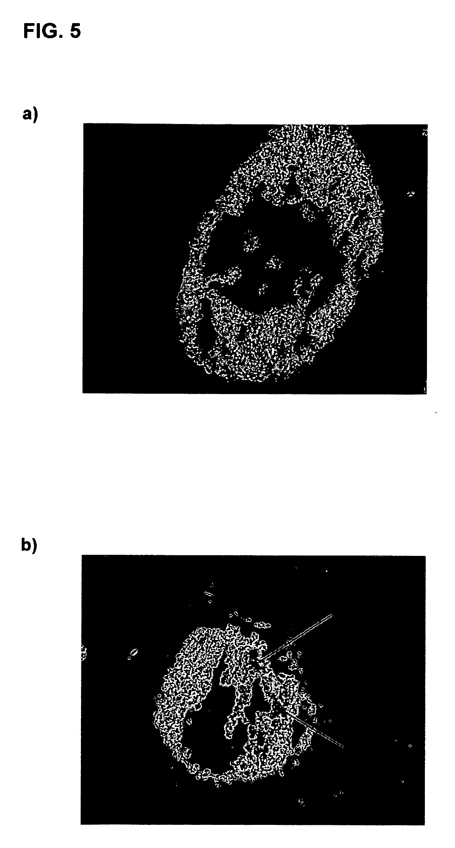

Figure 5 represents micrographs of stimulated cells according to the invention after chondrogenic stimulation which could be achieved by supplying the cells with 10 ng/ml TGFβ3. After 28 days of stimulation, cartilage-forming matrix can be detected by staining with toluidine blue (b) arrows). The treated cells show significant production of mucopolysaccharids (acidic glycosaminoglycans) indicating the successful differentiation.

As important components of the extracellular matrix, i.e. collagen type II and acidic glycosaminoglycans, could be detected these results demonstrate that the stem cells derived from certain soft tissue of tooth or wisdom tooth can be stimulated to differentiate by the method according to the invention and that these cells are at least capable to give rise to chondrocytes. For the first time, it could be demonstrated that stem cells derived from soft tissue located in immediate vicinity of teeth can be stimulated to differentiate into chondrocytes *in vitro.*

### References:

Pittenger, M.F., Mackay, A.M., Beck, S.C., Jaiswal, R.K., Douglas, R., Mosca, J.D., Moorman, M.A., Simonetti, D.W., Craig, S. and Marshak, D.R. (1999), Science 284, 143 - 147.

### List of reference numbers:

- 1: tooth
- 2: operculum
- 3: dental follicle
- 4: pad-like tissue
- 5: dental papilla
- 6: alveolar bone
- 7: extrapulpal soft tissue
- 8: border region

## Claims

1. Method for achieving differentiation of stem or progenitor cells derived from tooth tissue into chondrocytes *in vitro,* **characterised in that** said stem cells are isolated from a sleeve-like extrapulpal soft tissue of a tooth or wisdom tooth, wherein said stem cells are stimulated using Transforming Growth Factor β (TGFβ).

2. Method according to claim 1, **characterised in that** said stem cells are isolated from dental follicle of a tooth or wisdom tooth and/or a pad-like tissue located in immediate vicinity of the apical side of an immature, developing tooth or wisdom tooth underneath the dental papilla in a development stage between the appearance of the bony alveolar fundus and the end of the formation of the root of said tooth or wisdom tooth.

3. Method according to claim 1 or 2, **characterised in that** only stem cells are stimulated, which express at least surface marker CD90, CD49e and/or CD73, and/or which express surface marker CD13 at low level.

4. Method according to claim 1, 2 or 3, **characterised in that** only stem cells are stimulated, which do not exhibit any expression of surface marker CD31 and/or CD45.

5. Method according to any one of claims 1 to 4, **characterised in that** said stem cells are expanded in a cell culture medium comprising a minimal medium that contains at least 10 % foetal calf serum (FCS) and 1 g/l glucose.

6. Method according to any one of claims 1 to 5, **characterised in that** said stem cells are washed prior to stimulation with a cell culture medium comprising a minimal medium that contains at least 4.5 g/l glucose, 1 % ITS+ premix, 35 µg/ml L-ascorbate-2-phosphate, 1 mol/l sodium pyruvate and 10⁻⁷ mol/l dexamethasone.

7. Method according to any one of claims 1 to 6, **characterised in that** said stem cells are stimulated with TGFβ1 or preferably TGFβ3.

8. Method according to any one of claims 1 to 7, **characterised in that** stimulation of said stem cells is accomplished in a cell culture medium comprising a minimal medium that contains at least 4.5 g/l glucose, 1 % ITS+ premix, 35 µg/ml L-ascorbate-2-phosphate, 1 mol/l sodium pyruvate and 10⁻⁷ mol/l dexamethasone, preferably for at least 25 days, particularly 28 days.

9. Method according to any one of claims 1 to 8, **characterised in that** said tooth or wisdom tooth is a mammalian tooth, preferably a human tooth.

10. A differentiated former stem cell stimulated by the method according to any one of claims 1 to 9.

11. A Chondrocyte derived from a sleeve-like extrapulpal soft tissue of a tooth or wisdom tooth and/or dental follicle of a tooth or wisdom tooth and/or a pad-like tissue located in immediate vicinity of the apical side of an immature, developing tooth or wisdom tooth underneath the dental papilla, wherein said tooth or wisdom tooth is in a stage of development between the appearance of the bony alveolar fundus and the end of the formation of the root of said tooth or wisdom tooth:

12. The Chondrocyte according to claim 11, **characterised in that** said chondrocyte is a mammalian cell, preferably a human cell.

13. Cell culture or cell structure comprising at least one differentiated cell according to claim 10 and/or one chondrocyte according to claim 11 or 12.

14. Pharmaceutical composition comprising at least one differentiated cell according to claim 10 and/or one chondrocyte according to claim 11 or 12.

15. Use of the differentiated cell according to claim 10, or the chondrocyte according to claim 11 or 12, or the cell culture or cell structure of claim 13, or the pharmaceutical composition of claim 14, for repairing or replacing damaged or destroyed cartilage.

16. Use of the differentiated cell according to claim 10, or the chondrocyte according to claim 11 or 12, or the cell culture or cell structure of claim 13, or the pharmaceutical composition of claim 14, for treating arthrosis.

17. Use of at least one stem cell derived from a sleeve-like extrapulpal soft tissue of a tooth or wisdom tooth for obtaining differentiated chondrocytes.

18. Use of at least one stem cell derived from dental follicle of a tooth or wisdom tooth for obtaining differentiated chondrocytes.

19. Use of at least one stem cell derived from a pad-like tissue located in immediate vicinity of the apical side of an immature, developing tooth or wisdom tooth underneath the dental papilla in a development stage between the appearance of the bony alveolar fundus and the end of the formation of the root of said tooth or wisdom tooth for obtaining differentiated chondrocytes.
